(19) Europäisches Patentamt
European Patent Office
Office européen des brevets

(11) **EP 2 767 238 A1**

(12) **EUROPEAN PATENT APPLICATION**

(43) Date of publication:
20.08.2014 Bulletin 2014/34

(51) Int Cl.:
*A61B 6/06* (2006.01)  *A61B 6/14* (2006.01)
*A61B 6/00* (2006.01)  *G01N 23/04* (2006.01)
*A61B 6/03* (2006.01)

(21) Application number: 14154386.8

(22) Date of filing: 07.02.2014

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 13.02.2013 US 201313766709

(71) Applicant: Dental Imaging Technologies
Corporation
Hatfield, PA 19440 (US)

(72) Inventors:
• Elvin, Richard H.
Quakertown, PA Pennsylvania 18951 (US)
• Walsh, Edward S.
Media, PA Pennsylvania 19436 (US)
• Sebok, David A.
Eagleville, PA Pennsylvania 19403 (US)

(74) Representative: Garratt, Peter Douglas
Mathys & Squire LLP
The Shard
32 London Bridge Street
London SE1 9SG (GB)

(54) **Automatic field-of-view size calculation constraint**

(57) Systems and methods for field of view constraint. One system includes a processor. The processor is configured to receive a requested object field-of-view and determine if the object field-of-view is possible based on constraints of an imaging system, wherein the constraints include dimensions of a detector panel and motion limits of a collimator assembly. If the object field-of-view is possible, the processor is configured to dynamically generate a set of collimator motor commands and reconstruction parameters based on the object field-of-view, calibration parameters of the collimator assembly, and geometric calibration parameters of the imaging system. The processor is also configured to provide the set of collimator motor commands to the collimator assembly to position shutters of the collimator assembly to illuminate the object field-of-view.

FIG. 7

**Description**

FIELD

Embodiments of the invention relate to medical imaging systems, such as dental imaging systems. In particular, embodiments of the invention relate to systems and methods for optimizing collimation to provide a maximum field-of-view.

BACKGROUND

X-Ray images are acquired by projecting a source of X-rays through an object of interest onto a two-dimensional detector. Since the object will have a spatially-varying density, the resulting intensity pattern on the detector will be directly related to the density of the material in the object through which the various x-rays have passed. Thus, the resulting x-ray image is a representation of the internal structure of the object. X-ray imaging has particular application to medical imaging. X-ray imaging can be used to create a two-dimensional image or multiple two-dimensional images. Multiple two-dimensional images are created by rotating the x-ray source and the detector about an axis located within the object. The resulting images are processed to create a computed tomography ("CT") image data-set that provides three-dimensional information about the internal anatomy of the object.

SUMMARY

Optimal imaging characteristics occur when the detector is properly illuminated by x-rays. An important component in controlling the illumination is the x-ray collimator positioned between the x-ray source and the object being imaged. The collimator limits the extent of x-ray beams reaching the imaged object by blocking those x-rays that fall on the detector outside of a desired field-of-view ("FOV"). If the desired FOV is the entire detector, the objective would be to fully illuminate the detector while minimizing the number of x-rays that pass through the object but do not make it to the detector.

Since the detector can have significant cost, the goal would normally be to use and, therefore, illuminate as much of the detector as possible. However, radiating a significant area outside of the detector to ensure its full illumination increases the object's exposure to radiation.

In particular, conventional imaging systems use "protocol files" that define in a fixed form all of the scanning and reconstruction parameters needed for a particular FOV. These parameters include collimator motor settings for fully illuminating a particular part of the detector panel used to create images. The protocol files also include the geometry of the panel and the geometry of the reconstructed final volume. Accordingly, a limited number of protocols exist that are hand-crafted to optimize image quality. The protocols, however, were the same for all machines and did not take into account variations between the machines, such as panel size and collimator motion limits. To account for this deficiency, the protocols were conservative in that they irradiated the patient with more x-rays than needed for the requested FOV to ensure that the panel was properly illuminated.

There are additional practical issues associated with x-ray collimation. First, while the desired FOV and collimated x-ray beams are both generally rectangular, if there is any tilt in the collimator assembly, the useful FOV is defined by the horizontal rectangle that can fully fit within the tilted collimated x-ray rectangle. This issue can only be corrected by (1) increasing the collimated x-ray beam size and over-radiating the patient or (2) by shrinking the utilized FOV, which wastes expensive detector space. A second issue is that the finite diameter of the x-ray source means that the x-ray beam edges associated with the collimation edges are not sharp but rather gradually increases from zero to full intensity. The blurred shadow effect is also caused by a finite thickness of the collimator.

Both the degree of collimator tilt and the degree edge blurring vary on a system-by-system basis. As described above, conventional imaging systems account for these variations by assuming a worst case and under-collimating the patient (i.e., increase the x-ray beam size). However, this approach over-radiates the patient and does not effectively address system variations that fall outside a worse case expectation.

Accordingly, embodiments of the present invention provide systems and methods for optimizing collimation. In particular, the proposed systems and methods start with a desired image FOV and perform an automatic FOV size calculation constraint based on calibration measurements for both the overall imaging system geometry and the collimator assembly. The constraint is used to either fine tune the collimator assembly or, if the desired FOV cannot be obtained within the system constraints, indicate that a modified FOV is necessary. To perform fine-tuning of the collimator assembly, the degree of collimation is adjusted either by moving the collimator closer to or farther from the x-ray source or by changing the dimensions of the collimator aperture by moving one of more of the collimator edges. Therefore, the systems and methods make it possible to obtain optimal image quality on a system-by-system basis using a maximum amount of the detector panel while minimizing patient radiation exposure.

In particular, one embodiment of the invention provides a system for generating an image. The system includes a processor. The processor is configured to receive a requested object field-of-view and determine if the object field-of-view is possible based on constraints of an imaging system, wherein the constraints include dimensions of a detector panel and motion limits of a collimator assembly. If the object field-of-view is possible, the processor is configured to dynamically generate a set of collimator motor commands and reconstruction parameters based on the object field-of-view, calibration parameters of the collimator assembly, and geometric calibration parameters of the imaging system. The processor is also configured to provide the set of collimator motor commands to the collimator assembly to position shutters of the collimator assembly to illuminate the object field-of-view.

Another embodiment of the invention provides a method for generating an image. The method includes receiving a requested object field-of-view in a gantry space, converting, at a processor, dimensions of a detector panel to the gantry space based on geometric calibration parameters of an imaging system including the detector panel, and converting, at the processor, dimensions of a collimator assembly included in the imaging system to the gantry space based on the geometric calibration parameters and calibration parameters of the collimator assembly. The method also includes comparing the object field-of-view to the converted dimensions of the detector panel and the converted dimensions of the collimator assembly to determine if the object field-of-view is possible. If the object field-of-view is not possible, the method includes indicating to a user that a different object field-of-view be requested. If the object field-of-view is possible, the method includes converting, at the processor, the object field-of-view from the gantry space to a panel space based on the geometric calibration parameters and converting the object field-of-view from the panel space to a shutter space based on the calibration parameters of the collimator assembly. The method can also include acquiring image data based on the object field-view as converted to the shutter space.

Other aspects of the invention will become apparent by consideration of the detailed description and accompanying drawings.

BRIEF DESCRIPTION OF THE DRAWINGS

FIG. 1 illustrates a first medical image obtained using a prior-art medical imaging system.

FIG. 2 illustrates a second medical image obtained using a medical imaging system according to one embodiment of the present invention.

FIG. 3a illustrates a medical imaging system.

FIG. 3b illustrates a collimator assembly included in the medical imaging system of FIG. 3a.

FIG. 4 schematically illustrates the medical imaging system of FIG. 3a.

FIGS. 5a and 5b are a flow chart illustrating a method of optimizing collimation to provide a maximum field-of-view performed by the medical imaging system of FIG. 3a.

FIG. 6 graphically illustrates transformations of coordinates between various coordinate systems defined by the medical imaging system of FIG. 3a.

FIG. 7 schematically illustrates geometric calibration parameters for the medical imaging system of FIG. 3a.

FIG. 8 illustrates a matrix for transforming panel space coordinates to shutter space coordinates.

DETAILED DESCRIPTION

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways.

Also, it is to be understood that the phraseology and terminology used herein is for the purpose of description and should not be regarded as limiting. The use of "including," "comprising" or "having" and variations thereof herein is meant to encompass the items listed thereafter and equivalents thereof as well as additional items. The terms "mounted," "connected" and "coupled" are used broadly and encompass both direct and indirect mounting, connecting and coupling. Further, "connected" and "coupled" are not restricted to physical or mechanical connections or couplings, and can include electrical connections or couplings, whether direct or indirect. Also, electronic communications and notifications may be performed using any known means including direct connections, wireless connections, etc.

It should be noted that a plurality of hardware and software based devices, as well as a plurality of different structural components may be utilized to implement the invention. Furthermore, and as described in subsequent paragraphs, the specific configurations illustrated in the drawings are intended to exemplify embodiments of the invention and that other alternative configurations are possible.

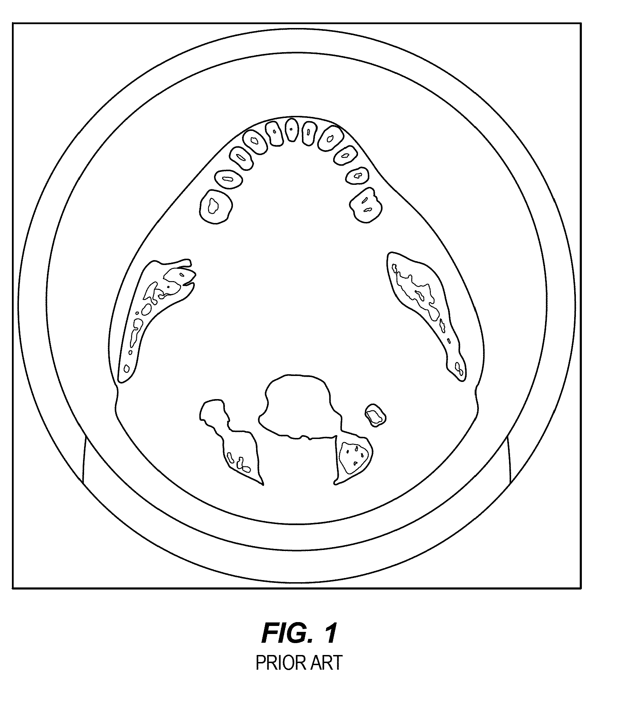

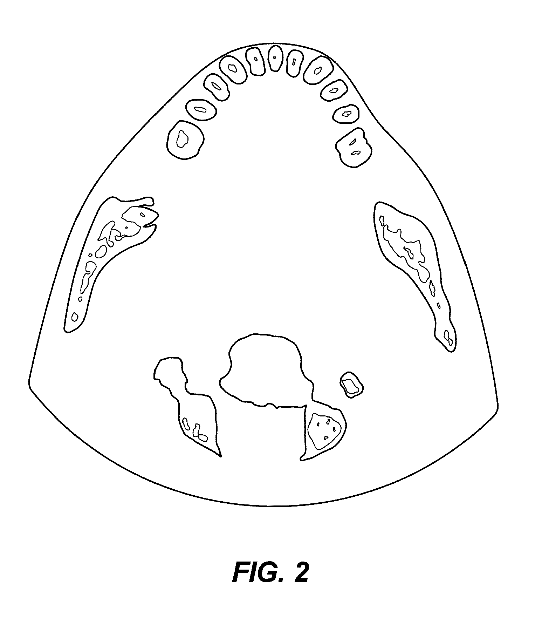

As described above, embodiments of the present invention optimize collimation to use a maximum area of a detector, which results in optimal image quality. For example, FIGS. 1 and 2 demonstrate the benefit of embodiments the invention on image quality. In particular, both images were acquired on the same machine but with different acquisition and reconstruction techniques. In both cases, an error in collimator dimensions was intentionally created that produces an illumination area on the detector panel that is approximately 5.0 millimeters from the edge of the detector on all four sides. FIG. 1 illustrates an image generated using a prior-art CT acquisition and image reconstruction. FIG. 2 illustrates an image generated using the systems and methods of the present invention. In particular, with respect to the image illustrated in FIG. 1 and as described in more detail below, a desired field-of-view ("FOV") was combined with system geometric calibrations and collimator calibrations to produce a system-based correction for the collimator position. As illustrated in FIG. 1, the image generated using the prior-art systems and methods is darker than the image illustrated in FIG. 2. In addition, unlike the image illustrated in FIG. 2, the image illustrated in FIG. 1 contains a bright, dark ring around its outer circumference.

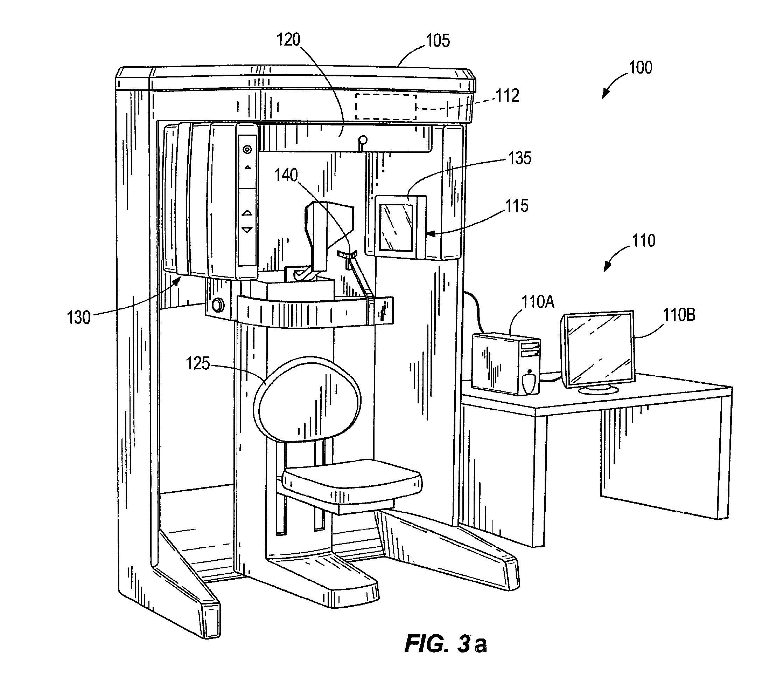

FIG. 3a illustrates a medical imaging system 100 for generating an image as illustrated in FIG. 2. The system 100 includes an imaging apparatus 105 and a workstation 110. The imaging apparatus 105 includes a scanner that scans an object. The workstation 110 includes a computer 110A and a touchscreen 110B. In some embodiments, the computer 110A and the touchscreen 110B are combined in a single device. Also, in some embodiments, the workstation 110 includes peripheral devices, such as a keyboard, mouse, printer, etc., connected to the computer 110A and/or the touchscreen 110B. In addition, it should be understood that in some embodiments, a non-touch-sensitive screen is used in place of or in addition to the touchscreen 110B.

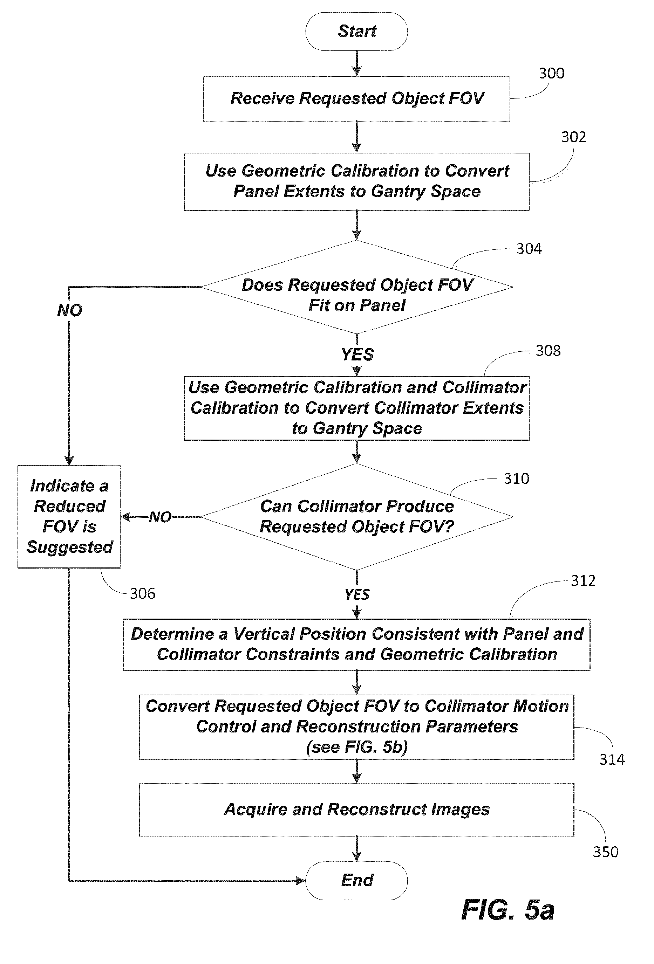

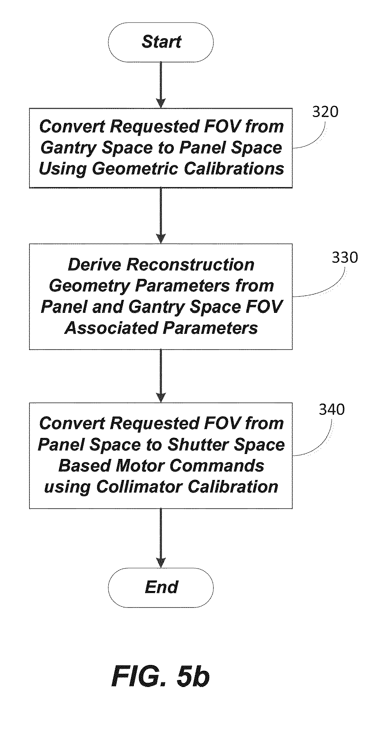

As described in more detail below with respect to FIGS. 5a and 5b, the computer 110A is configured to (1) determine if a requested object FOV is possible given system constraints, (2) constrain the requested object FOV with optimized collimator and reconstruction parameters for optimize image quality, (3) instruct the imaging apparatus 105 to perform a scan of an object based on the constrained FOV, (4) receive data generated by the imaging apparatus 105 during the scan, and (5) reconstruct one or more images based on the acquired data. The computer 110A can be connected to the imaging apparatus 105 by one or more wired or wireless connections. In some embodiments, the computer 110A is also configured to display at least one of the reconstructed images on the touchscreen 110B. It should be understood that the functionality of the computer 110A described in the present application can be distributed among multiple computers in various configurations. For example, some of the functionality of the computer 110A can be performed by a computer included in the imaging apparatus 105.

The imaging apparatus 105 is, for example, a dental CT device and includes an on-board computer or processor 112, a radiation detector 115, a gantry 120, a support 125 for an object or patient being imaged, and a radiation source 130. The radiation detector panel 115 is positioned on the gantry 120 opposite the radiation source 130 and includes a detector array 135 having a plurality of detection elements. During a scan, a patient sits on the support 125 (and places his or her chin in a chin support 140). The gantry 120 is rotated around the patient's head, and, as the gantry 120 rotates, the radiation source 130 moves and directs radiation at the patient's head at various angles. The radiation detector 115 detects the radiation passing through the patient and generates a data set including three-dimensional data.

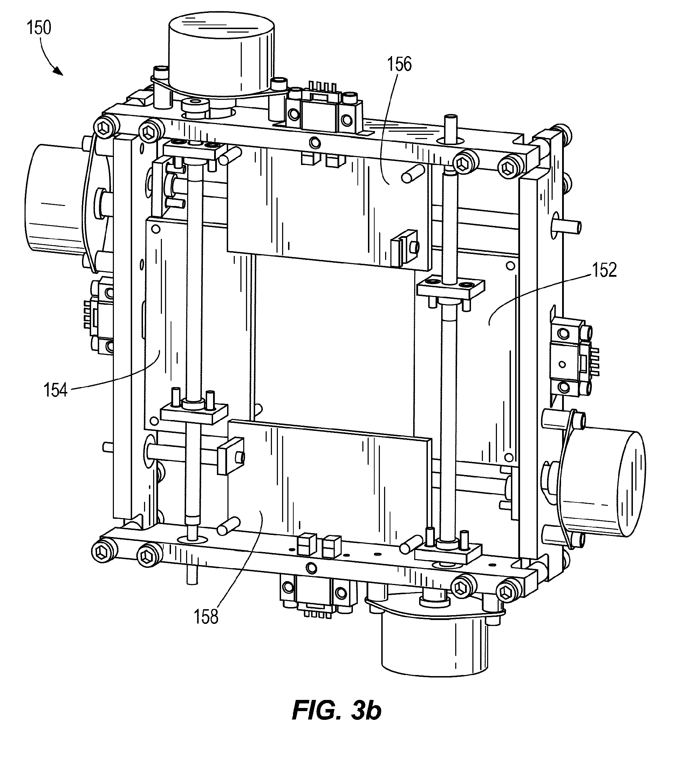

Positioned between the radiation source 130 and the patient, is a collimator assembly. As illustrated in FIG. 3b, the collimator assembly 150 includes four shutters: a right shutter 152, a left shutter 154, a top shutter 156, and a bottom shutter 158. When illuminated by radiation, the shutters 152, 154, 156, and 158 block a portion of the radiation, which casts a shadow on the detector panel 115. The shadow, accordingly, encroaches on the imaging area from the right, left, top, and bottom. The collimator assembly 150 also includes at least one motor assembly that allows for independent control of each shutter. Therefore, each shutter 152, 154, 156, and 158 can be positioned independently of the other shutters.

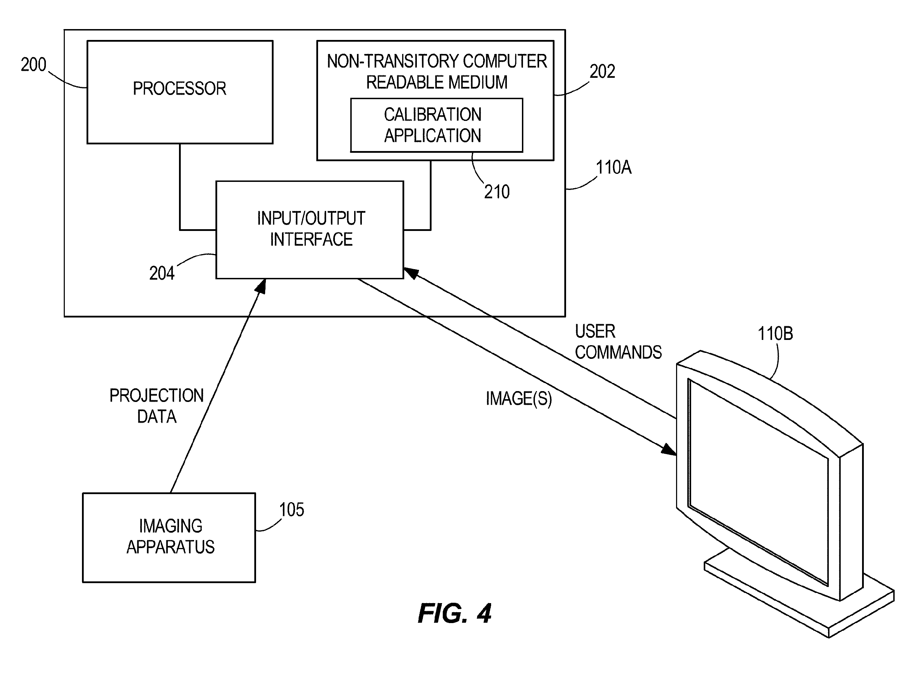

As illustrated in FIG. 4, the computer 110A is connected to the imaging apparatus 105 and the touchscreen 110B. The computer 110A includes a processor 200, non-transitory computer-readable medium 202, and an input/output interface 204. It should be understood that in other constructions, the computer 110A includes additional, fewer, or different components. The processor 200 is configured to retrieve instructions and data from the media 202 and execute, among other things, the instructions to receive data from the imaging apparatus 105 and output data to the touchscreen 110B (i.e., generate a signal for displaying data on the touchscreen 110B).

The input/output interface 204 transmits data from the processor 200 to external systems, networks, and/or devices and receives data from external systems, networks, and/or devices. In particular, the input/output interface 204 communicates with the imaging apparatus 105 and the touchscreen 110B over one or more wired or wireless connections and/or networks. The input/output interface 204 can also store data received from external sources to the media 202 and/or provide the data to the processor 200.

The computer-readable media 202 stores program instructions and data including a calibration application (or "application") 210. As described in more detail below with respect to FIGS. 5a and 5b, when executed by the processor 200, the application 210 calculates an automatic FOV size calculation constraint based on calibration measurements for both the system geometry and the collimator assembly 150.

As illustrated in FIG. 4, a user uses the system 100 to initiate a CT scan with the imaging apparatus 105, which generates projection data (i.e., set of x-ray projection frames, plus the positions of the x-ray source 130 and the x-ray detector 115 for each projection frame). The computer 110A receives the projection data and uses the data to generate three-dimensional, volumetric data, which can be used by the computer 110A to render images displayed to a user on the touchscreen 110B or another display.

In particular, the processor 200 included in the computer 110A executes the calibration application 210 (or a separate user interface application) to display various screens to a user on the touchscreen 110B. A user enters commands and image generation settings through the displayed screens using buttons on the screens (selectable through the touchscreen 110B itself or separate peripheral devices, such as a keyboard or a mouse) to initiate a scan. To initiate a scan, a user selects a patient and one or more image generation settings. The image generation settings include an object FOV. The object FOV defines the diameter and height in gantry space of an area of the object that the user wants to image. As illustrated below in Table 1, the imaging system 100 defines a plurality of coordinate systems based on various components of the system 100 and raster images generated by the system. For example, the gantry space includes a two-dimensional coordinate system defined with respect to the center of rotation of the gantry 120 and directly relatable to the patient.

Table 1: Coordinate Systems

| Coordinate System | Abbreviation | Units | Origin | Description |
|---|---|---|---|---|
| Shutters | $S_{RLTB}$ | Steps | Open = 0 | 1D Line of motion x Four Shutters |
| Gantry | G | (mm) | Center Beam | 2D Cartesian, device FOV centric |
| Panel | P | (mm) | Panel midpoint | 2D Cartesian, panel centric |
| Raster | R | Pixels | Upper Left | 2D Cartesian, raster projection image |

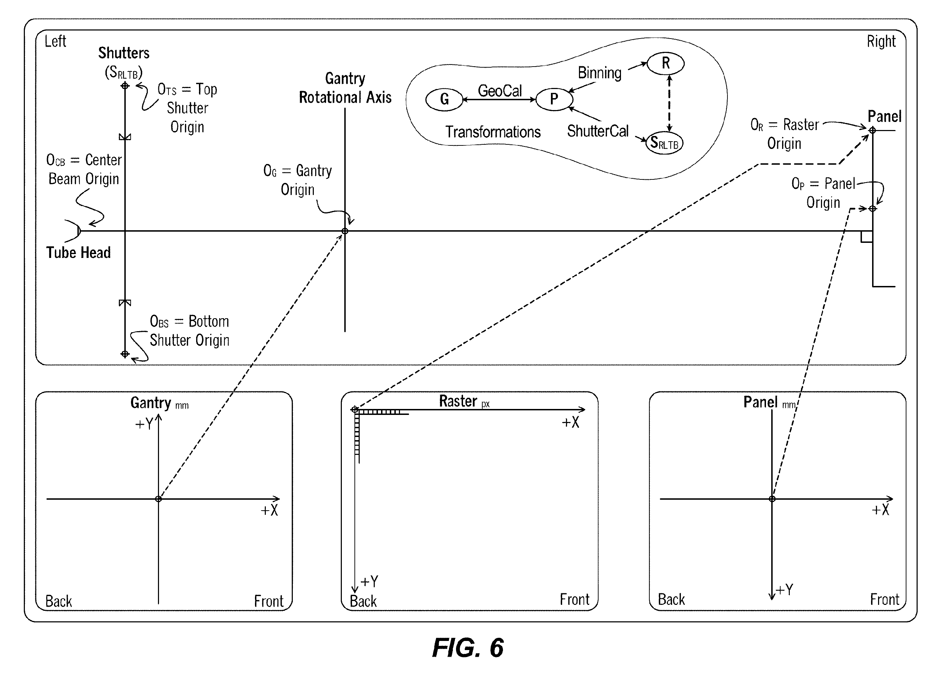

As illustrated in FIG. 5a, the application 210 receives the object FOV (at step 300). Next, the application 210 determines whether the requested object FOV will fit on the detector panel 115. In particular, because the object FOV is specified in gantry space, the application 210 first converts dimensions of the detector panel 115 to gantry space using the geometric calibration parameters for the imaging system 100 (at step 302). First, as illustrated in FIG. 6, coordinates can be transformed between various coordinate systems, such as from panel space to gantry space. However, geometric calibration parameters and collimator calibration parameters for the system 100 are needed to perform the transformations.

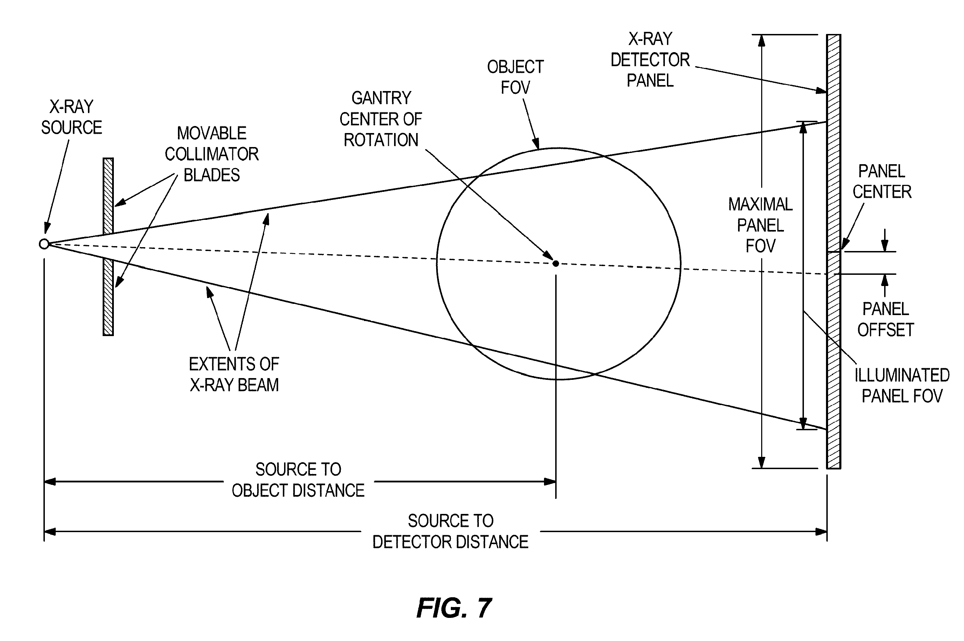

Geometric calibration parameters for the system 100 are typically used during image reconstruction, which requires knowledge of the exact geometry of the imaging system 100. In particular, the following parameters are generally important for image reconstruction and are generally illustrated in FIG. 7. A source-to-object-distance parameter represents a distance from the radiation source 130 to a center of rotation of the gantry 120. A source-to-detector-distance parameter represents a distance from the radiation source 130 to the detector panel 115 passing through the center of rotation of the gantry 120. A panel-horizontal-offset parameter represents a horizontal offset distance between the physical center of the detector panel 115 and an x-ray beam passing from the radiation source 130 to the detector panel 115 through the center of rotation of the gantry 120. A panel-vertical-offset parameter represents a vertical offset distance between the physical center of the detector panel 115 and an x-ray beam passing from the radiation source 130 to the detector panel 115 through the center of rotation of the gantry 120. A panel-pivot parameter represents an angle between the detector panel's center vertical line and a true scanner vertical defined by the rotation axis of the gantry. A gantry-full-rotation parameter represents an actual angle of gantry rotation associated with approximately 360 degrees of planned scanner rotation.

It should be understood that other geometric calibration parameters exist but may not be used by the application 210 because they have a negligible effect on image quality. As is generally known, geometric calibration of the imaging system 100 can be accomplished by scanning an object containing structures that are easily visible or identifying on x-ray images (e.g., as spherical balls) and that have known dimensions and relationship. A full set of projection images are obtained from the calibration object. The location of the structures on the images are then obtained and processed to determine the set of geometric calibration parameter values most consistent with the relationship between the physical location of the structures in the scanned object and the location of the structures in the projection images.

Using the geometric calibration parameters, the application 210 can convert the extents (i.e., dimensions, such as diameter and height) of the detector panel 115 to gantry space dimensions. In particular, the application 210 can determine a maximal panel FOV (see FIG. 7) in gantry space. The application 210 then compares the extents of the detector panel 115 (transformed to gantry space) to determine if the object FOV (i.e., the diameter and height) falls within the extents of the detector panel 115 (at step 304, FIG. 5a). The object FOV is already in gantry space, so it need not be converted. Thus, the comparison of the detector panel edges and the object FOV is conducted in gantry space. If the object FOV (which is already in gantry space) does not fall within the extents of the panel 115 (as transformed to gantry space), the application 210 may indicate to the user that a reduced object FOV is suggested (at step 306). Accordingly, the user can reinitiate the scan at a reduced object FOV to enhance image quality and better utilize the system components (e.g., the detector panel).

Alternatively, if the object FOV fits on the detector panel 115, the application 210 determines if the collimator assembly 150 can generate the object FOV. In particular, the application 210 uses the collimator calibration parameters to convert the extents of the collimator assembly (e.g., motion limits) to panel space and then uses the geometric calibration parameters to convert collimator extents to gantry space (at step 308). The application 210 then compares, in gantry space, the converted collimator extents to the object FOV to determine if the collimator assembly 150 can produce the requested object FOV (at step 310). If the collimator assembly 150 cannot generate the requested object FOV (e.g., the motion limits of the collimator assembly 150 prevent the collimator from illuminating the entire object FOV), the application 210 may indicate to the user that a reduced object FOV is suggested (at step 306). As noted above, in this situation, a user can reinitiate the scan with a reduced object FOV.

Calibration of the collimator assembly 150 determines where the edge of the x-ray beams will fall on the detector panel 115 for a particular shutter's motor setting. In particular, collimator or shutter calibration tries to determine the relationship between a shutter motor's settings and the actual position of the edge of the shadow generated by the shutter on the detector panel 115. There are multiple ways to calibrate the collimator assembly 150. One way includes acquiring a set of raster images with the collimator shutters at various locations. In each image, the location and nature of the top, bottom, left, and right edges of the x-ray beam are characterized in terms of slopes and intercepts. Once the respective slopes and intercepts at each shutter location have been identified, the information is processed to produce the following parameters for all four shutters (see Table 2 below). These parameters are characterized in a shutter space (referred to as "$S_{RLTB}$" since it involves right, left, top, and bottom edges) that is defined by a one-dimensional line of motion of a shutter (i.e., with the exception of the shutter-function-slopes, which are characterized as a ratio of the panel and shutter coordinate system).

Table 2: Collimator Calibration Parameters

| Attribute | Coordinate System | Units | Description |
|---|---|---|---|
| Shutter Edge Steps | $S_{RLTB}$ | Steps | The number of shutter steps needed for locating the shutter at the matching panel edge. |
| Shutter Error Intercepts | $S_{RLTB}$ | Steps | Maximum shutter alignment correction with perpendicular shutter cropping at zero. |
| Shutter Error Slopes | $S_{RLTB}$ / $S_{RLTB}$ | Cropped Steps/ Steps | Rate alignment error increases per perpendicular cropping decrease. |
| Shutter Function Intercepts | $S_{RLTB}$ | Steps | The number of shutter steps need for locating the shutter at panel center. |
| Shutter Function <u>Slopes</u> | $O_P/S_{RLTB}$ | (mm) / Steps | Number of shutter steps per millimeter. |

If the collimator assembly 150 can produce the requested object FOV, the application 210 proceeds to set up the scan for the requested object FOV. In particular, as described in more detail below, the object FOV is converted to panel space and collimator space to set up the collimator assembly 150 for the scan and set corresponding reconstruction parameters that optimize use of the detector panel 115 while minimizing patient radiation exposure (e.g., optimized collimator and reconstruction parameters). As illustrated in FIG. 5a, one step of this set up process includes determining a vertical position consistent with panel constraints and the geometric calibration parameters (at 312). The initial FOV chosen by the user includes vertical top and bottom positions in gantry space. When these positions are transformed to the panel space, the positions emanate from a panel center. However, the bottom shutter's requested distance from the panel center may not satisfy the lower half of the vertical size requested in the initial FOV. Therefore, if this occurs, it may be desirable to automatically adjust the vertical position of the FOV by adjusting (i.e., shifting up by a small amount) the top and bottom positions in order to adapt to collimator constraints.

The application 210 also converts the requested object FOV to collimator motion control and reconstruction parameters (at 314). In particular, as illustrated in FIG. 5b, the application 210 converts the object FOV from gantry space dimensions to panel space dimensions using the system geometric calibration parameters (at 320). In particular, the gantry space is defined as a two-dimensional coordinate system based on the location of a center beam generated by the radiation source 120 ($G_{(mm)}$). The panel space is defined as a two-dimensional coordinate system based on a midpoint of the detector panel 115. In particular, a given gantry-space distance $G_{(mm)}$ included in the object FOV is transformed into a panel-space distance $P_{(mm)}$ as follows:

$$(1) \quad P_{(mm)} = \text{Translate}( \text{Rotate} ( \text{FlipY} ( \text{Scale} ( G_{(mm)} ) ) ) )$$

$$(2) \quad \text{Scale}_{(mm)} = \text{Gantry}_{(mm)} * \text{Zoom}$$

$$(3) \quad \text{Zoom} = \text{DSD} / \text{DSO}$$

$$(4) \quad \text{DSO} = \text{geometric calibrated distance from radiation source to object}$$

$$(5) \quad \text{DSD} = \text{geometric calibrated distance from source to detector}$$

$$(6) \quad \text{FlipY}_{(mm)} = \text{Scale}_{(mm)} * -1 \quad \text{(for top and bottom shutters only)}$$

$$(7) \quad \text{Rotate}_{(mm)} = \text{Rotate}(\Theta, \text{FlipY}_{(mm)})$$

$$(8) \quad \Theta = \text{Detector Pivot}$$

$$(9) \quad \text{Translate}_{(mm)} = \text{Rotate}_{(mm)} + \text{Offsets}_{(mm)}$$

$$(10) \quad \text{Offsets}_{(mm)} = \text{Detector Offsets}_{(mm)}$$

In particular, the above equations (1) through (10) illustrate how the parameters determined during the calibration process are used to mathematically convert gantry-space coordinates to panel-space coordinates. For example, equation (1) indicates that to get panel coordinates in millimeters, a scaling is initially applied to gantry coordinates in millimeters to account for the larger shadow on the panel than the actual object (i.e., Scale ( $G_{(mm)}$ )), the y-coordinate axis direction of the result of the scaling step is then flipped (i.e., FlipY ( Scale ( $G_{(mm)}$ )), the result of the flipping step is then rotated to account for detector pivot (i.e., Rotate ( FlipY (Scale ( $G_{(mm)}$ )))), and the result of the rotating step is then translated to account for the horizontal and vertical offset of the panel midpoint (i.e., Translate( Rotate ( FlipY ( Scale ( $G_{(mm)}$ )))).

With regard to the scaling step, equation (2) indicates that multiplying the gantry coordinates by a "Zoom" factor, defined in equations (3) through (5), results in scaled values. With regard to the flipping step, equation (6) indicates that the coordinates resulting from the scaling step are negated because the panel space y-axis points down instead of up. With regard to the rotating step, equation (4) indicates that rotated coordinates in millimeters can be obtained by rotating the results of the flipping step by a predetermined degree (i.e., theta degrees) defined in equation (8). It should be noted that the use of the term "Rotate" with the subscript (mm) in the above equations indicates coordinates that result from the rotation operation, whereas the term "Rotate" without the subscript indicates the rotation operation itself. Finally, with respect to the translating step, equation (9) indicates that adding offsets to the result of the rotation step provides translated coordinates. Equation (10) indicates that the offsets used in equation (9) are those produced by the geometric calibration process.

Thus, as illustrated in the above equations (1) through (10), gantry coordinate space values in millimeters can be transformed to panel space using five output parameters of geometric calibration (i.e., pivot, DSD, DSO, horizontal and vertical offset) and standard coordinate system transformations (i.e., scale, flip, rotate, and translate).

The application 210 derives reconstruction parameters based on the object FOV panel space parameters or dimensions and the object FOV gantry space parameters (at 330). In particular, to dynamically create the imaging protocol for optimal image quality, both machine parameters (e.g., collimator motor commands, as described below) and reconstruction parameters need to be adjusted. For example, the application 210 dynamically adjusts reconstruction parameters such as edge cropping, input pixel size, output volume size, and output volume positions when dynamically creating the scanning and associated reconstruction protocol. This creates a reconstructed image volume of size and position consistent with the requested FOV size and position.

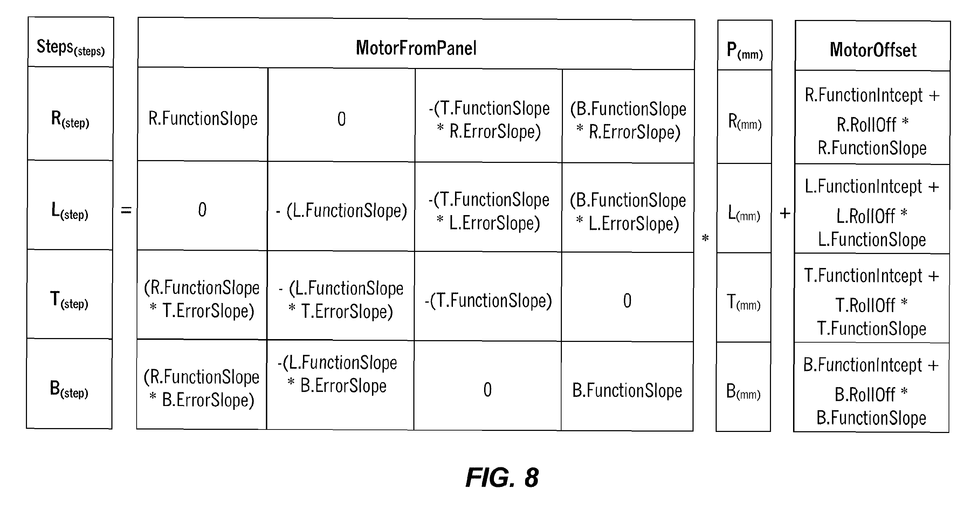

The application 210 also converts the object FOV from panel space to shutter-space-based motor commands using the collimator calibration parameters (at 340). In particular, the application 210 uses the above collimator calibration parameters to convert the object FOV dimensions from the panel space $P_{(mm)}$ to shutter-space-based-motor-commands in the shutter space. In some embodiments, the application 210 uses a matrix as illustrated in FIG. 8 based on the following equation to calculate all of the $S_{RLTB\,(Steps)}$ positions, given the input $P_{(mm)}$ coordinates.

$$(11) \qquad S_{RLTB\,(Steps)} = MotorFromPanel * P_{(mm)} + MotorOffset$$

With the coordinates converted to the shutter space, the application 210 activates the top, bottom, left, and right collimator motors to properly position each collimator shutter based on the converted coordinates. With the collimator positioned, the imaging apparatus 105 radiates the object to acquire one or more images of the object (at step 350, FIG. 5a), such as the image illustrated in FIG. 2.

Thus, embodiments of the present invention provide systems and methods for dynamically creating parameters associated with a scanning protocol. Accordingly, the systems and methods can theoretically create an infinite number of protocols that use collimation to illuminate with x-rays only the portion of a patient necessary to obtain quality images. In addition, the dynamically-created parameters adapt to particular system characteristics and conform to non-ideal attributes of the system. Accordingly, even imaging systems far from ideal produce quality images. In general, the dynamic systems and methods assume that a system consists of "constraints" such as panel dimensions and collimator motion limits and "characteristics" (calibration parameters), such as source-to-object-distance, panel-tilt, panel-offset, and collimator motor calibration. Therefore, given a user requested FOV size and location, the system uses the constraints and characteristics to determine if the desired FOV is possible. If it is possible, the systems and methods use the characteristics to dynamically create a set of collimator motor commands and reconstruction parameters to perform image data acquisition and image reconstruction.

Various features and advantages of the invention are set forth in the following claims.

## Claims

1. A system for generating images, the system comprising:

   a processor configured to:

   receive a requested object field-of-view;
   determine if the object field-of-view is possible based on constraints of an imaging system, the constraints including dimensions of a detector panel and motion limits of a collimator assembly;
   if the object field-of-view is possible, dynamically generate a set of collimator motor commands and reconstruction parameters based on the object field-of-view, calibration parameters of the collimator assembly, and geometric calibration parameters of the imaging system; and
   provide the set of collimator motor commands to the collimator assembly to position shutters of the collimator assembly to illuminate the object field-of-view.

2. The system of Claim 1, wherein the object field-of-view is defined in a gantry space.

3. The system of Claim 1 or Claim 2, wherein the processor is configured to determine if the object field-of-view is possible by converting the dimensions of the detector panel to the gantry space based on the geometric calibration parameters.

4. The system of Claim 3, wherein the processor is configured to determine if the object field-of-view is possible by determining if the object field-of-views fits within the converted dimensions of the detector panel in the gantry space.

5. The system of Claim 1, wherein the processor is configured to determine if the object field-of-view is possible by converting the dimensions of the collimator assembly to the gantry space based on the geometric calibration parameters and the collimator calibration parameters.

6. The system of Claim 5, wherein the processor is configured to determine if the object field-of-view is possible by determining if the object field-of-views fits within the converted dimensions of the collimator assembly in the gantry space.

7. The system of any one of the preceding claims, wherein the processor is further configured to indicate to a user that a reduced field-of-view is suggested if the object field-of view is not possible.

8. The system of any one of the preceding claims, wherein the processor is configured to dynamically generate the set of collimator motor commands and the reconstruction parameters by converting the object field-of-view to a panel space based on the geometric calibration parameters, deriving the reconstruction parameters from the object field-of-view in the gantry space and in the panel space, and convert the object field-of-view to a shutter space based on the geometric calibration parameters and the calibration parameters of the collimator assembly to generate the set of collimator motor commands.

9. The system of any one of the preceding claims, further comprising a collimator assembly receiving said collimator motor commands to position shutters of the collimator assembly.

10. A method of generating an image, the method comprising:

receiving a requested object field-of-view in a gantry space;
converting, at a processor, dimensions of a detector panel to the gantry space based on geometric calibration parameters of an imaging system including the detector panel;
converting, at the processor, dimensions of a collimator assembly included in the imaging system to the gantry space based on the geometric calibration parameters and collimator calibration parameters of the collimator assembly;
comparing the object field-of-view to the converted dimensions of the detector panel and the converted dimensions of the collimator assembly to determine if the object field-of-view is possible;
if the object field-of-view is not possible, indicating to a user that a different object field-of-view be requested;
if the object field-of-view is possible, converting, at the processor, the object field-of-view from the gantry space to a panel space based on the geometric calibration parameters and converting the object field-of-view from the panel space to a shutter space based on the calibration parameters of the collimator assembly; and
acquiring image data based on the object field-view as converted to the shutter space.

11. The method of Claim 10, further comprising generating reconstruction parameters based on the object field-of-view as converted to the panel space and using the reconstruction parameters to construct an image based on the image data.

12. The method of Claim 10 or Claim 11, further comprising displaying an image based on the image data.

**FIG. 1**
PRIOR ART

FIG. 2

FIG. 3a

EP 2 767 238 A1

**FIG. 3b**

*FIG. 4*

Start

Receive Requested Object FOV — 300

Use Geometric Calibration to Convert Panel Extents to Gantry Space — 302

Does Requested Object FOV Fit on Panel — 304

NO

YES

Use Geometric Calibration and Collimator Calibration to Convert Collimator Extents to Gantry Space — 308

Indicate a Reduced FOV is Suggested

Can Collimator Produce Requested Object FOV? — 310

NO

306

YES

Determine a Vertical Position Consistent with Panel and Collimator Constraints and Geometric Calibration — 312

Convert Requested Object FOV to Collimator Motion Control and Reconstruction Parameters (see FIG. 5b) — 314

Acquire and Reconstruct Images — 350

End

FIG. 5a

Start

Convert Requested FOV from Gantry Space to Panel Space Using Geometric Calibrations — 320

Derive Reconstruction Geometry Parameters from Panel and Gantry Space FOV Associated Parameters — 330

Convert Requested FOV from Panel Space to Shutter Space Based Motor Commands using Collimator Calibration — 340

End

# FIG. 5b

**FIG. 6**

FIG. 7

| Steps$_{(steps)}$ | | MotorFromPanel | | | | | P$_{(mm)}$ | | MotorOffset |
|---|---|---|---|---|---|---|---|---|---|
| R$_{(step)}$ | = | R.FunctionSlope | 0 | -(T.FunctionSlope * R.ErrorSlope) | (B.FunctionSlope * R.ErrorSlope) | * | R$_{(mm)}$ | + | R.FunctionIntcept + R.RollOff * R.FunctionSlope |
| L$_{(step)}$ | | 0 | - (L.FunctionSlope) | -(T.FunctionSlope * L.ErrorSlope) | (B.FunctionSlope * L.ErrorSlope) | | L$_{(mm)}$ | | L.FunctionIntcept + L.RollOff * L.FunctionSlope |
| T$_{(step)}$ | | (R.FunctionSlope * T.ErrorSlope) | - (L.FunctionSlope * T.ErrorSlope) | -(T.FunctionSlope) | 0 | | T$_{(mm)}$ | | T.FunctionIntcept + T.RollOff * T.FunctionSlope |
| B$_{(step)}$ | | (R.FunctionSlope * B.ErrorSlope) | -(L.FunctionSlope * B.ErrorSlope | 0 | B.FunctionSlope | | B$_{(mm)}$ | | B.FunctionIntcept + B.RollOff * B.FunctionSlope |

**FIG. 8**

EP 2 767 238 A1

Europäisches
Patentamt

European
Patent Office

Office européen
des brevets

# EUROPEAN SEARCH REPORT

**Application Number**

EP 14 15 4386

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2006 311882 A (GE MED SYS GLOBAL TECH CO LLC) 16 November 2006 (2006-11-16) | 1,9 | INV. A61B6/06 |
| Y | * abstract * * paragraph [0026] - paragraph [0028] * | 2,3,5,7,8 | A61B6/14 A61B6/00 |
| A | * paragraph [0032] - paragraph [0037] * * paragraph [0044] - paragraph [0051] * * figures 1,3,6,7 * | 10-12 | G01N23/04 ADD. A61B6/03 |
| Y | JP 2009 136425 A (HITACHI MEDICAL CORP) 25 June 2009 (2009-06-25) | 2,3,5,7,8 | |
| A | * abstract * * paragraph [0012] - paragraph [0046] * * figures 1,7,8,11 * | 1,9-12 | |
| A | US 2003/179851 A1 (ISHIKAWA TAKAYUKI [JP]) 25 September 2003 (2003-09-25) * abstract * * paragraph [0024] - paragraph [0033] * * figures 2,3 * | 1-12 | |
| A | US 2012/243662 A1 (LOUSTAUNEAU VINCENT [FR] ET AL) 27 September 2012 (2012-09-27) * abstract * * paragraph [0078] - paragraph [0159] * * figures 1-2,4-5 * | 1,2,7,9,10,12 | TECHNICAL FIELDS SEARCHED (IPC) A61B G01N G06T |
| A | WO 2013/011403 A1 (KONINKL PHILIPS ELECTRONICS NV [NL]; HEROLD MARK DOUGLAS [US]) 24 January 2013 (2013-01-24) * abstract * * page 4, paragraph 4 - page 11, paragraph 4 * * figures 7-16 * | 1,2,9,10,12 | |
| A | US 2003/165216 A1 (WALKER MATTHEW J [US] ET AL KUMP KENNETH S [US] ET AL) 4 September 2003 (2003-09-04) * the whole document * | 1,7,10,12 | |

The present search report has been drawn up for all claims

| Place of search | Date of completion of the search | Examiner |
|---|---|---|
| Munich | 7 April 2014 | Artikis, T |

EPO FORM 1503 03.82 (P04C01)

**ANNEX TO THE EUROPEAN SEARCH REPORT
ON EUROPEAN PATENT APPLICATION NO.**

EP 14 15 4386

This annex lists the patent family members relating to the patent documents cited in the above-mentioned European search report.
The members are as contained in the European Patent Office EDP file on
The European Patent Office is in no way liable for these particulars which are merely given for the purpose of information.

07-04-2014

| Patent document cited in search report | | Publication date | Patent family member(s) | | Publication date |
|---|---|---|---|---|---|
| JP 2006311882 | A | 16-11-2006 | NONE | | |
| JP 2009136425 | A | 25-06-2009 | NONE | | |
| US 2003179851 | A1 | 25-09-2003 | CN | 1454569 A | 12-11-2003 |
| | | | JP | 2003230555 A | 19-08-2003 |
| | | | US | 2003179851 A1 | 25-09-2003 |
| US 2012243662 | A1 | 27-09-2012 | EP | 2512346 A1 | 24-10-2012 |
| | | | FR | 2953707 A1 | 17-06-2011 |
| | | | JP | 2013514111 A | 25-04-2013 |
| | | | KR | 20120114267 A | 16-10-2012 |
| | | | US | 2012243662 A1 | 27-09-2012 |
| | | | WO | 2011080460 A1 | 07-07-2011 |
| WO 2013011403 | A1 | 24-01-2013 | CN | 103648393 A | 19-03-2014 |
| | | | WO | 2013011403 A1 | 24-01-2013 |
| US 2003165216 | A1 | 04-09-2003 | DE | 10309268 A1 | 18-09-2003 |
| | | | FR | 2836738 A1 | 05-09-2003 |
| | | | JP | 2003284708 A | 07-10-2003 |
| | | | US | 2003165216 A1 | 04-09-2003 |

EPO FORM P0459

For more details about this annex : see Official Journal of the European Patent Office, No. 12/82